# EUROPEAN PATENT APPLICATION

(11) **EP 1 717 236 A1**
(43) Date of publication of application: **02.11.2006**
(21) Application number: 05103539.2
(22) Date of filing: 28.04.2005
(51) Int. Cl.: C07D 319/12

(54) **Method for purifying glycolide**

(71) Applicant: Purac Biochem B.V., 4200 AA Gorinchem (NL)
(72) Inventor: Uitslag, Tom John, 4205 LN Gorinchem (NL); Van Krieken, Jan, 4207 WN Gorinchem (NL); Krul, Raymon Reinier, 2613 VS Delft (NL)
(74) Representative: Beetz, Tom

(57) **Abstract**

The invention pertains to a method for purifying glycolide comprising the step of distilling crude glycolide under reflux using a distillation column having a ratio S/CxL > 1.1 and S²/C²L > 0.4 m, wherein
S stands for the inner surface of the column in m²;
L x C stands for the outer surface of the column in m², wherein
L stands for the length of the column in m; and
C stands for the average circumference of the column in m,
to obtain glycolide having a purity expressed as a turbidity < 10 NTU as measured by the method according to DIN 27027 /ISO 7027 of a solution of 1 g of said glycolide in 5 ml of acetone.

## Description

The present invention relates to a method for purifying glycolide.

Glycolide is a known cyclic dimer ester of glycolic acid, and is usually obtained by polymerizing glycolic acid to a product containing oligomers of glycolic acid, followed by depolymerization by heating the glycolic acid oligomers, which reaction is carried out continuously or batch-wise.

The present invention is also concerned with glycolide, which is reduced or substantially eliminated from adverse influences ascribable to impurities contained in commercially obtainable glycolide to meet the purity requirements of users thereof.

Polyglycolic acid is a polyester formed by dehydration- polycondensation of glycolic acid (i.e., α-hydroxyacetic acid) and containing repeat unit according to the following formula:
- (O-CH2-CO)n-
wherein n is commonly 2-100, preferably 2-30.

Glycolide is a cyclic ester compound having the structure wherein two water molecules are eliminated from two glycolic acid molecules having the following formula:

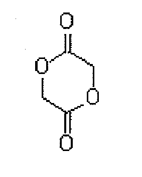

Only by the esterification reaction of glycolic acid, however, glycolide cannot be obtained because of the formation of glycolic acid oligomers. So far, various glycolide production processes have been proposed. For instance, in US Pat. No. 2,668,162 a process is disclosed in which a glycolic acid oligomer is crushed into powders and heated at 270 to 285° C under an ultra-high vacuum. Alternatively, US Pat. Nos. 4,835,293 and 5,023,349 teach a process wherein polyglycolic acid oligomer is heated into a melt, and glycolide is generated and vaporized out of the surface of the melt and is entrained in an inert gas such as nitrogen gas and stripped in a low- boiling solvent such as acetone or ethyl acetate for recovery. Other processes wherein a glycolic acid oligomer is heated in a polar organic solvent having a high boiling point, and distilling out the resultant glycolide together with the polar organic solvent, and removing the glycolide from the distillates, are also known.

Glycolide has many applications, for instance for ring-opening polymerization in the presence of a catalyst such as stannous octoate to obtain high molecular weight polymers (also called "polyglycolide").

To produce polyglycolide having a high molecular weight by the ring-opening polymerization of glycolide, it is required to use high-purity glycolide as the starting material. To use glycolide as the starting material to produce polyglycolic acid on an industrial scale, it is thus essential to economically feed such high- purity glycolide.

In a common procedure glycolide is tested by dissolving glycolide in acetone. A stubborn problem by doing so is the occurrence of turbid or hazed solutions. Such hazed solutions commonly have a milk-like appearance, which is a strong indication that the glycolide is unsuitable for many applications. It is therefore an object of the present invention to provide a method for obtaining glycolide that on dissolving in acetone provides a clear solution, free from any opacification or turbidity.

To this end a method for purifying glycolide was found comprising the step of distilling crude glycolide under reflux using a distillation column having a ratio S/CxL > 1.1 and S²/C²L > 0.4 m, wherein
S stands for the inner surface of the column in m²;
L x C stands for the outer surface of the column in m², wherein
L stands for the length of the column in m; and
C stands for the average circumference of the column in m, to obtain glycolide having a purity expressed as a turbidity < 10 NTU as measured by the method according to DIN 27027 /ISO 7027 of a solution of 1 g of said glycolide in 5 ml of acetone.

The glycolide for use in the purification method of the invention can be glycolide produced by any process including depolymerization of a glycolic acid oligomer for instance by heating a glycolic acid oligomer or a glycolic acid oligomer plus a polar organic solvent to depolymerize the glycolic acid oligomer into glycolide. By way of example but not by way of limitation, the depolymerization method usable herein includes melt depolymerization as set forth typically in U.S. Pat. No. 2,668,162, solution depolymerization as set forth typically in U.S. Pat. No. 5,830,991 and JP-A 09-328481, and solid- phase depolymerization as set forth typically in JP-A 11-116666.

As the depolymerization reaction system comprising a mixture of a glycolic acid oligomer and a polar organic solvent is heated, the glycolide resulting from the depolymerization reaction and the polar organic solvent are co-distilled out. By separating the glycolide from distillates, the glycolide may be recovered. In this case, too, however, without using the purification method of the invention the glycolide is insufficiently free from contaminants to prevent the occurrence of haze when dissolved in acetone.

It was found that regular distillation did not give glycolide of sufficient purity to prevent haze-forming when dissolved in acetone. The common practice for improving the distillation efficiency, i.e. using longer distillation columns did not lead to significant improvement. Surprisingly it was now found that short distillation columns could give excellent results when these columns are provided with a large inner surface. Thus if a hollow cylindrical tube is used as distillation column the inner surface is substantially the same as the outer surface. In a Vigreux column the inner surface is increased by means of extensions provided in the wall of the tube. In this case the inner surface is larger than the outer surface (which is still calculated as would it be a perfect cylinder). The term "outer surface" denotes the theoretical surface, not taking into account irregularities. Thus if the tube is a cylinder having a circumference C, the outer surface is C x L, wherein L is the length of the tube. In case this same tube is a Vigreux column, the outer surface is still the same, irrespective from possible extra surface that could be present due to the inwardly directed extensions. In case the tube is not a perfect cylinder, for instance having sections with different circumferences, the average circumference is used. For a perfect cylinder the average circumference is the same as the circumference. For any tube, whether it is cylindrical, ellipsoidal, rectangular, hexagonal, and the like, the surface is correctly calculated by multiplying the length of the tube by the circumference thereof. It is further clear to the skilled person of how to calculate the outer surface of distillation columns. If a plurality of columns is used, the values in the above formulas are those of the plurality of columns.

Any Vigreux column satisfying the formula S/CxL > 1.1 and S²/C²L > 0.4 m can be used to obtain glycolide of the required purity. S/CxL is a measure for the difference of the inner and outer surface of the column. S²/C²L is a measure for the relation between the volume of the column and the difference of the inner and outer surface of the column. To this end Vigreux columns may be used. However, in order to use columns that are as short as possible it is advantageous to use columns having much higher ratios, preferably S/CxL > 2. Such high ratio is practically impossible for Vigreux columns. To obtain such high ratio columns the columns are filled with an inert material having a large surface (i.e., materials that are not affected by glycolide, other chemical compounds present, solvents used and temperature) such a glass, ceramics, steel, and the like. Examples of such materials are, for instance, rings, saddles, beads, fibers and the like. However, although these materials are in principle suitable for purifying glycolide, clogging or slow performance may become a serious problem when the density of these materials increases too much. Therefore structured column packings, such as available at Sulzer Chemtech, Switzerland (Sulzer packing) or poured packings, are preferred to increase the inner surface of the distillation column These packings contribute significantly to the column inner surface S, thus increase the ratio S/CxL considerably. They can be made of various materials, including stainless steels, carbon steel, copper-bronze, monel, hastelloy, nickel, titanium, aluminum, brass, plastic (PP, PVDF, Teflon® PFA), glass, ceramics, and the like.

It was found that such columns, particularly columns filled with structural packing, in most instances suffice for purifying glycolide. In those cases where further improvement is required, for instance if short columns or columns having a relatively low S/CxL ratio are used, the glycolide vapor can be cooled in the upper part of the distillation column, i.e. in the upper half part of the column, more preferably at or close at the top of the distillation column. Such cooling can be obtained by using a cooling element such as a cooling spiral that can be cooled with water. This additional cooling element provides an increased liquid flux, apparently thereby stripping impurities from the gaseous glycolide.

In another aspect of the invention there is provided in a distillation installation for purifying glycolide comprising a distillation column having a ratio S/CxL > 1.1 and S²/C²L > 0.4 m, wherein
S stands for the inner surface of the column;
L stands for the length of the column; and
C stands for the average circumference of the column.

Preferably, this distillation installation has a distillation column, which is filled with a column packing, more preferably a structured column packing. In a particularly preferred embodiment the distillation installation is provided at its upper part with a cooling element.

In preferred embodiments the invention pertains to columns having S/CxL > 2, more preferably > 3, most preferably > 5.

Preferred ratios S²/C²L are > 2 m, more preferably > 5 m, and most preferably > 10 m.

The term "reflux" has the common meaning, i.e. the situation that in the distillation column the downwardly directed liquid stream is in contact and in equilibrium (steady state) with the upwardly directed vapor stream.

The invention is illustrated by the following non-limitative examples.

### General

Turbidity instruments measure the average volume of light scattering over a defined angular range. The particle size and the concentration of suspended solids, as well as the level of dissolved solids can affect the reading. Turbidity is defined as an expression of the optical property that causes light to be scattered and absorbed, rather than transmitted, in straight lines through the sample, i.e. turbidity is the measure of relative sample clarity.

The following data were obtained with a WTW Turb 550 IR (WTW Wissenschaftlich-Technische Werkstätten GmbH, Germany).

Turbidity instruments can measure turbidity and the amount of suspended solids. Turbidity is measured as Nephelometric Turbidity Units (NTU), which represents the average volume scattering over a defined angular range. The particle size and concentration of suspended solids as well as dissolved solids can affect this reading. When measuring suspended solids, the instruments measure the concentration expressed in parts per million (ppm).

The solutions according to the invention should have a turbidity < 10 NTU, preferably < 5 NTU, more preferably <3.5 NTU, even more preferably <2 NTU, and most preferably < 1 NTU.

The following distillation experiments were performed to demonstrate the purification method.

### Example 1

1856 g of glycolic acid pre-polymer were heated in a 2 l round bottom flask to 231° C under a pressure of 8 mbar. The distillation column consisted of an unfilled cylindrical column with S/CxL = 1 and S²/C²L = 0.15 m. The glycolide obtained showed a severe haze when dissolved in acetone (1 g of glycolide in 5 ml of acetone).

### Example 2

1854 g of glycolic acid pre-polymer were heated in a 2 l round bottom flask to 233° C under a pressure of 4 mbar. The distillation column consisted of a Vigreux with S/CxL = 1.3 and S²/C²L = 0.5 m. The glycolide obtained showed no haze (< 10 NTU) when dissolved in acetone (1 g of glycolide in 5 ml of acetone).

### Example 3

1774 g of glycolic acid pre-polymer were heated in a 2 l round bottom flask to 240° C under a pressure of 4 mbar. The distillation column consisted of a cylindrical column (length 0.60 meter; diameter 0.025 meter) partially filled with 2 Sulzer packings (type EX) (height 0.055 meter each) with S/CxL = 1.6 and S²/C²L = 4.1 m. The glycolide obtained showed no haze (< 10 NTU) when dissolved in acetone (1 g of glycolide in 5 ml of acetone).

### Example 4

37.7 kg of glycolic acid pre-polymer were heated in a 30 1 stainless steel reactor to 230° C under a pressure of 4 mbar in a semi-continuous manner. The distillation column consisted of a cylindrical column (length 1 meter; diameter 0.10 meter) partially filled with 2 Sulzer packings (type DX, height 0.055 meter each) with S/CxL = 2.4 and S²/C²L = 5.6 m. The glycolide obtained showed no haze (< 10 NTU) when dissolved in acetone (1 g of glycolide in 5 ml of acetone).

## Claims

1. A method for purifying glycolide comprising the step of distilling crude glycolide under reflux using a distillation column having a ratio S/CxL > 1.1 and S²/C²L > 0.4 m, wherein
S stands for the inner surface of the column in m²;
C x L stands for the outer surface of the column in m², wherein
L stands for the length of the column in m; and
C stands for the average circumference of the column in m,
to obtain glycolide having a purity expressed as a turbidity < 10 NTU as measured by the method according to DIN 27027 / ISO 7027 of a solution of 1 g of said glycolide in 5 ml of acetone.

2. The method according to claim 1, wherein the ratio S/CxL > 2, preferably >3.

3. The method according to claim 1 or 2, wherein the ratio S²/C²L > 2 m, preferably > 5 m.

4. The method according to any one of claims 1 to 3, wherein the distillation is performed in a column filled with a column packing.

5. The method according to claim 4, wherein the distillation is performed in a column filled with a structured column packing.

6. The method according to any one of claims 1 to 5, wherein the glycolide in its vapor phase is cooled in the upper part of the distillation column.

7. The method according to claim 6 wherein the vapor is cooled at or close at the top of the distillation column.
